# NOUVEAU FASCICULE DE BREVET EUROPEEN

(11) **EP 0 711 743 B2**
(45) Date de publication et mention de la décision concernant l'opposition: **11.01.2006**
(45) Mention de la délivrance du brevet: 30.06.1999
(21) Numéro de dépôt: 95402491.5
(22) Date de dépôt: 07.11.1995
(51) Int. Cl.: C07C 31/26, C12P 7/58, C07C 29/141, C07C 29/88, A61Q 11/00

(54) **Compositions de polyols, son procédé de préparation et ses applications**
Polyol-Zusammensetzungen, Verfahren zu deren Herstellung und deren Anwendungen
Polyol compositions, process for their preparation and their applications

(30) Priorité: 10.11.1994 FR 9413583; 06.06.1995 US 467320
(43) Date de publication de la demande: 15.05.1996
(73) Titulaire: Roquette Frères, 62136 Lestrem (FR)
(72) Inventeur: Lefevre, Philippe, F-59600 Merville (FR); Salome, Jean-Paul, F-59232 Vieux-Berquin (FR)
(74) Mandataire: Boulinguiez, Didier

(56) Documents cités:
- EP-A- 0 168 315
- FR-A- 2 054 829
- FR-A- 2 177 931
- JP-A- 6 379 844

## Description

La présente invention a trait à une nouvelle composition de polyols présentant une très grande stabilité thermique et une très grande stabilité chimique en milieu alcalin et une très faible réactivité.

Elle concerne également un procédé de préparation de cette nouvelle composition ainsi que ses applications, en particulier son utilisation pour la préparation de dentifrices.

On désigne par le terme polyols, dans la présente invention, les produits obtenus par hydrogénation catalytique de sucres réducteurs simples, mais aussi de sucres réducteurs plus complexes composés des homologues supérieurs de ces sucres simples, tels que les disaccharides, oligosaccharides et polysaccharides ainsi que leurs mélanges.

Généralement, les sucres réducteurs simples que l'on destine à l'hydrogénation catalytique pour l'obtention des compositions de polyols du type de celles de l'invention sont le glucose, le xylose, le fructose et le mannose. Les polyols obtenus sont alors le sorbitol, le xylitol et le mannitol.

Les disaccharides sont le plus souvent le maltose, l'isomaitulose, le maltulose, l'isomaltose et le lactose, qui conduisent, par hydrogénation, au maltitol, à l'isomalt, à l'isomaltitol et au lactitol.

Les oligosaccharides et polysaccharides, qui sont des produits de poids moléculaire plus élevé, proviennent d'ordinaire d'une hydrolyse acide et/ou enzymatique d'amidons et/ou de fécules, de xylans ou de fructans comme l'inuline, mais peuvent aussi être obtenus par recombinaison acide et/ou enzymatique de mono ou de disaccharides tels que ceux cités plus haut.

Par composition de polyols, on entend dans la présente invention les mélanges de polyols qui forment des sirops incristallisables à 20°C et à une matière sèche de 70 % lorsqu'ils sont conservés dans un récipient hermétique à l'air durant un mois de stockage. Certains de ces sirops peuvent même former des verres organiques, comme des sucres cuits, résistants à la cristallisation.

Les industries de la confiserie, de la pharmacie, de l'hygiène bucco-dentaire et même les industries chimiques utilisent couramment des compositions de polyols dans la fabrication, par exemple, de bonbons cuits sans sucre, de sirops anti-acides ou antitussifs, de pâtes dentifrices, de mousses de polyuréthane.

Les sirops de sorbitol obtenus par hydrogénation de glucose ou d'hydrolysats d'amidon de richesse en glucose variable mais élevée, représentent une classe très importante en raison de leur poids économique. Ces sirops sont en premier lieu utilisés en tant que produits de remplacement des sucres. Leur goût sucré permet de préparer de nombreux produits peu caloriques et peu ou non cariogènes telles que certaines confiseries ou tels que certains sirops pharmaceutiques.

Les sirops de sorbitol sont utilisés en second lieu en raison de leur excellent pouvoir humectant. De telles utilisations sont largement décrites dans: "LE SORBITOL", F. Baudart, Ed. DUNOD (1971), p. 19 à 75. C'est ainsi le cas dans la fabrication de dentifrices, de crèmes et de laits cosmétiques, de mousses à raser mais aussi de produits alimentaires comme les viennoiseries et les pâtisseries et d'autres produits comme le tabac ou le papier. Dans ces domaines, on utilise de préférence des sirops de sorbitol ou plutôt des compositions de polyols non cristallisables ou difficilement cristallisables car c'est la propriété d'humectance, seule conférée à l'état de solutés, qui est recherchée.

En troisième lieu, d'autres utilisations des sirops de sorbitol mettent à profit les propriétés plastifiantes du sorbitol. C'est le cas dans l'industrie des colles, des plastiques biodégradables, ou encore dans l'industrie du chewing-gum.

En quatrième lieu, enfin, les sirops de sorbitol sont parfois utilisés en tant qu'intermédiaires chimiques dans la fabrication par exemple d'esters de sorbitan ou d'initiateurs pour la fabrication de mousses de polyuréthane et de résines alkydes. Dans ce cas, c'est aux propriétés chimiques des polyols que l'on fait appel.

Les sirops de maltitol, obtenus par hydrogénation catalytique d'hydrolysats d'amidon de richesse variable en maltose, représentent également une classe importante parmi les compositions de polyols. Ils sont aujourd'hui essentiellement utilisés en raison de leur pouvoir sucrant élevé pour préparer, comme à l'aide de sirops de sorbitol, des produits alimentaires et pharmaceutiques non cariogènes. Il a été envisagé de les utiliser également en tant qu'intermédiaires chimiques pour préparer des tensioactifs et entrer dans la composition de mousses de polyuréthane.

Les sirops de xylitol, représentant une troisième classe des compositions de polyols, sont à ce jour des produits en plein développement. Bien que plus coûteux que les sirops de sorbitol ou de maltitol, on songe à les utiliser dans les mêmes applications que celles citées plus haut en raison notamment de leur pouvoir sucrant très élevé et de leurs excellentes propriétés humectantes.

L'industrie emploie également d'autres compositions de polyols. Des sirops de glucose hydrogénés riches en oligo et polysaccharides, trouvent ainsi application dans le domaine de la fonderie, de la trempe des métaux ou de la détergence. De telles applications sont décrites dans les brevets FR 2 348 771 et EP 100 280.

D'autres sirops à base d'oligo et de polysaccharides hydrogénés, obtenus par recombinaison acide et/ou enzymatique de glucose ou de maltose notamment, commencent à être utilisés dans l'industrie. Il s'agit par exemple du polydextrose hydrogéné dont on s'accorde à penser qu'il pourrait être avantageusement employé dans l'industrie alimentaire, comme agent de charge très peu calorique, en particulier dans la fabrication de boissons, de produits glacés et de confiseries. Ceci est décrit en particulier dans les brevets US 3 766 165 et US 3 876 794.

Il est connu que les applications des compositions de polyols sont cependant limitées dans certains secteurs de l'industrie par le fait que ces compositions ne satisfont pas pleinement aux contraintes de stabilité thermique et de stabilité en milieu alcalin ou de réactivité à l'égard de quelques substances spécifiques (Dunod, p. 28).

On observe ainsi un phénomène de jaunissement à haute température de ces compositions lorsqu'elles sont utilisées dans la fabrication de sucres cuits. Une telle coloration est souvent incompatible avec certaines aromatisations des bonbons.

Dans la fabrication des pâtes dentifrices alcalines, pâtes dans lesquelles des cristaux de carbonate ou de phosphate de sodium sont utilisés, on préfère employer d'autres produits pourtant moins économiques que les compositions de polyols, tels que les glycols, les propanediols, ou la glycérine. Car, les compositions de polyols prennent dans ces produits une coloration brune dans le temps.

Dans la fabrication de certains tabacs de qualité, on préfère aussi utiliser la glycérine en raison des goûts parasites que confèrent les compositions de polyols, bien que leurs propriétés humectantes soient considérées comme supérieures.

Il arrive aussi qu'on écarte ces compositions de polyols de la fabrication des tensio-actifs, des mousses de polyuréthane, des lessives, des détergents et des sirops pharmaceutiques anti-acides en raison de la couleur jaunâtre, voire brune, qu'ils confèrent à ces produits, bien que là encore, ils satisfassent à toutes les autres contraintes techniques relatives à ces applications (DUNOD, p.75).

Il existe donc à l'heure actuelle un besoin de disposer de compositions de polyols thermiquement et chimiquement plus stables que celles disponibles sur le marché.

Or, il est du mérite de la Société demanderesse d'avoir découvert que des compositions de polyols constituées de mélanges de polyols qui forment des sirops incristallisables présentent des conditions de stabilité satisfaisantes dès lors que leur densité optique, mesurée dans un test S, est intérieure ou égale à 0,100.

Le test S, repose sur une mesure spectrophotométrique appliquée aux produits à tester.

Pour conduire ce test S, on procède comme suit:
- on amène, si besoin est, par concentration ou par dilution aqueuse le sirop de polyol à tester à une matière sèche de 40 % en poids,
- on ajoute à 5ml de celle solution 500mg d'hydrogénocarbonate de sodium de qualité ultrapure, vendu par exemple sous le nom RP NORMAPUR™ pour analyses par la société PROLABO, 65 Bd Richard Lenoir, PARIS, FRANCE, et 250mg d'une solution aqueuse à 20 % d'ammoniac,
- on mélange l'ensemble et on le chauffe pendant 2 heures au bain-marie à 100°C sans imposer d'agitation,
- on amène la solution à 20°C et on mesure la densité optique de la solution ainsi obtenue à une longueur d'onde de 420 nm grâce à un spectrophotomètre tel que celui commercialisé par PERKIN-ELMER sous la marque Lambda 5 UV/VIS Spectrophotometer. Grâce à cet appareil, à titre d'exemple, on obtient des densités optiques de 0,040, 0,080 et 0,120 en remplaçant les 5 ml de solution de polyols à 40 % de matière sèche par 5 ml d'une solution contenant respectivement 40, 80 et 120 parties par million de D⁺ glucose anhydre RP NORMAPUR™ pour analyses (société PROLABO), dissous dans de l'eau distillée.

La composition de polyols est d'autant plus stable que la valeur mesurée dans le test S est basse.

Comme l'a vérifié la demanderesse, il faut noter que, de façon surprenante et inattendue, il ne semble pas y avoir de correspondance entre d'une part la richesse des compositions de polyols en un polyol particulier, ou encore leur teneur en sucres réducteurs résiduels ou libres (mesurée selon la méthode usuelle cupro-tartro-sodique de Bertrand ou la méthode faisant appel à l'acide dinitrosalicylique) et le résultat obtenu dans le test S d'autre part.

Autrement dit, il ne semble pas exister de lien direct entre la stabilité thermique et chimique d'une composition de polyols et sa teneur en sucres réducteurs résiduels.

Ceci pourrait s'expliquer par la fait que par le test S on obtient une mesure globale et que la coloration obtenue dans ce test dépend probablement tout à la fois du pH final du sirop, de la quantité de minéraux présents au sein du sirop, de la nature de ces minéraux, de la quantité de fonctions réductrices non réduites par l'hydrogénation, ainsi que de la nature des molécules portant ces fonctions non réduites : monosaccharides, disaccharides, oligo ou polysaccharides.

Ainsi, par exemple, on observe dans le test S que de façon surprenante, des traces en oligo- et polysaccharides non réduits génèrent, toutes choses étant égales par ailleurs, des colorations plus intenses que ne le font des mono- et des disaccharides non réduits, à une même concentration de fonctions réductrices.

C'est ainsi que 180 parties par million (ppm) de dextrose sont moins sujets à une coloration en milieu basique que 342 ppm de maltose exprimant le même pouvoir réducteur, qui eux-mêmes colorent moins que des concentrations supérieures d'oligosaccharides ou de polysaccharides exprimant le même pouvoir réducteur.

Une telle constatation amène à penser qu'il serait nécessaire et suffisant, afin d'obtenir des compositions de polyols conformes à l'invention -surtout lorsqu'elles contiennent beaucoup d'oligo et polysaccharides réduits- de poursuivre plus longtemps que de coutume l'hydrogénation catalytique, de manière à obtenir des teneurs en sucres réducteurs résiduels ou libres à la limite de la détection analytique.

L'hydrogénation catalytique du glucose ou des sirops de glucose, de même que celle du fructose ou du xylose, telle qu'elle est couramment pratiquée, est décrite par exemple dans "CHEMICAL CONVERSION OF STARCH BASED GLUCOSE SYRUPS, chap 9, pages 278-281, de "Starch Conversion Technology", 1985, vol 14 par A.P.G KIEBOOM et H. VAN BEKKUM.

La société demanderesse a cependant remarqué que d'une part il n'était pas nécessaire de prolonger de façon irraisonnable l'hydrogénation catalytique afin d'obtenir la composition selon l'invention et que d'autre part une telle prolongation de l'hydrogénation ne permettait pas d'obtenir ladite composition.

Il apparaît en effet que les di-, oligo-, et polysaccharides sont plus difficiles à hydrogéner totalement que les monosaccharides.

Au terme de nombreux travaux, la demanderesse a trouvé qu'il convenait, afin d'obtenir les compositions conformes à l'invention, d'ajouter au moins une étape supplémentaire dite de "stabilisation" aux procédés classiques d'hydrogénation catalytique. Cette étape de stabilisation peut consister, par exemple et sans que cela ne soit limitatif, en une étape de fermentation, d'oxydation, ou de caramélisation.

L'étape de stabilisation permet d'obtenir une composition de polyols présentant une densité optique inférieure ou égale à 0,100 dans le test S. Cette étape doit être placée après l'étape d'hydrogénation et de préférence avant l'ultime étape de purification de la composition de polyols.

L'invention se rapporte donc en premier lieu à l'utilisation pour la préparation de dentifrices d'une composition de polyols constituée de mélanges de polyols qui forment des sirops incristallisables et caractérisée en ce qu'elle en ce qu'elle présente une densité optique inférieure ou égale à 0,100 dans le test S. De préférence, la composition de polyols utilisée selon l'invention présentant une densité optique inférieure ou égale à 0,100 dans le test S, comprend, par rapport à sa teneur en polyols à l'état sec, une teneur de 0,01 à 95 % de monosaccharides hydrogénés et/ou de disaccharides hydrogénés, le complément à 100 % des polyols étant constitué par des oligo et polysaccharides hydrogénés, ces teneurs étant exprimées par rapport à la matière sèche de polyols présents.

Les monosaccharides hydrogénés peuvent être choisis avantageusement dans le groupe comprenant le sorbitol, l'iditol, le mannitol, le xylitol, l'arabitol et l'érythritol, et plus préférentiellement parmi le sorbitol, le mannitol et le xylitol.

Les disaccharides hydrogénés peuvent être choisis avantageusement dans le groupe comprenant le maltitol, le maltulose hydrogéné, l'isomaltulose hydrogéné ou isomalt (mélange de glucopyranosido-1,6-mannitol et de glucopyranosido-1,6-sorbitol), l'isomaltitol, le lactitol, l'inulobiose hydrogéné, et plus préférentiellement parmi le maltitol, le lactitol et l'isomaltulose hydrogéné.

Les oligosaccharides et polysaccharides hydrogénés peuvent être constitués par le maltotriitol, le maltotétraitol et autres oligo- et polysaccharides hydrogénés obtenus par hydrolyse de l'amidon suivie d'une hydrogénation. Lesdits oligosaccharides et polysaccharides hydrogénés peuvent cependant être également constitué par le cellobiitol, le cellotriitol, le xylobiitol, le xylotriitol et autres oligo- et polysaccharides hydrogénés obtenus par hydrolyse, généralement acide, de cellulose, de xylans, de fructans comme par exemple l'inuline, de dextrines, de polyglucoses comme le polydextrose, suivie d'une hydrogénation. Lesdits oligo- et polysaccharides hydrogénés peuvent aussi provenir d'une recombinaison acide ou enzymatique de mono- ou disaccharides, éventuellement réduits, tels que ceux cités ci-dessus, seuls ou en présence d'autres oligo- et polysaccharides éventuellement réduits, suivie d'une hydrogénation. Les oligosaccharides et polysaccharides hydrogénés que l'on préfère voir présents au sein de la composition, sont les oligo- et polysaccharides issus d'hydrolysats hydrogénés d'amidons, de dextrines et les polyglucoses hydrogénés, éventuellement hydrolysés au préalable.

La teneur en mono- et disaccharides de la composition de polyols conforme à l'invention est plus préférentiellement comprise entre 0.1 et 90 %, plus préférentiellement encore entre 0.5 et 86 % et mieux encore entre 50 et 86 % des polyols, lesdites teneurs étant exprimées par rapport à la matière sèche des polyols présents dans la composition ; la teneur en oligo- et polysaccharides hydrogénés représentant le complément à 100 % de cette matière sèche. C'est ainsi qu'on peut obtenir avantageusement, pour certaines applications, une composition moins sujette à la cristallisation de l'un ou l'autre de ces mono- ou disaccharides hydrogénés.

De façon à convenir au mieux aux emplois où les contraintes en terme de goût, de stabilité thermique ou chimique sont les plus draconiennes, la composition de polyols constituée de mélanges de polyols qui forment des sirops incristallisables utilisée conformément à l'invention présente de préférence une densité optique inférieure ou égale à 0,075, plus préférentiellement inférieure ou égale à 0,060, et mieux encore inférieure ou égale à 0,040 dans le test S. On peut noter ici que ces valeurs de densité optique, qui caractérisent la composition de polyols conforme à l'invention, sont très nettement inférieures aux valeurs trouvées pour les compositions de polyols décrites ou commercialisées à ce jour. En effet pour ces demiers produits la densité optique dans le même test S, est toujours très supérieure à 0,100 et généralement comprise en 0,500 et 0,850.

La composition de polyols constituée de mélanges de polyols qui forment des sirops incristallisables utilisée conformément à l'invention, selon qu'elle est plus ou moins riche en mono- et disaccharides, présente une teneur en sucres totaux après hydrolyse totale selon la méthode de Bertrand, comprise entre 3,5 et 98 %, de préférence comprise entre 6 et 92 % et plus préférentiellement encore comprise entre 8 et 90 %, cette teneur étant exprimée par rapport à la matière sèche de la composition.

La composition de polyols constituée de mélanges de polyols qui forment des sirops incristallisables utilisée conformément à l'invention peut être présentée sous forme de sirop ou de poudre, selon la destination ultérieure qui lui est réservée. Elle peut également, compte tenu de sa très grande stabilité et de sa haute compatibilité à la très grande majorité des ingrédients ou additifs utilisés dans l'industrie, être mélangée à des produits les plus divers. En particulier, on peut sans aucun inconvénient ajouter à la composition de l'invention des glycols comme l'éthylène glycol, le propylène glycol, le dipropylène glycol, le diéthylène glycol et les polyéthylène glycols, de la glycérine ou des propanediols pour ajuster les propriétés fonctionnelles de la composition ou pour la présenter sous forme d'un sirop à très haute matière sèche, c'est-à-dire jusqu'à 96 % de matière sèche.

L'invention se rapporte en second lieu à un procédé de préparation d'une composition stable de polyols.

Ce procédé est caractérisé en ce que l'on soumet un sirop de polyols obtenus par hydrogénation catalytique de sucres réducteurs simples ou complexes à la succession des étapes suivantes :
- une étape de stabilisation telle qu'une fermentation, une oxydation ou une caramélisation, visant à amener la densité optique du sirop hydrogéné à une valeur inférieure ou égale à 0,100, de préférence inférieure ou égale à 0,075 et plus préférentiellement encore inférieure ou égale à 0.060 dans le test S.
- une étape de purification du sirop hydrogéné "stabilisé" ainsi obtenu.

Il va de soi que le procédé conforme à l'invention peut comprendre d'autres étapes classiques et connues de l'homme du métier comme en particulier des étapes de purification sur terres, charbon actif et/ou résines, de concentration et de séchage éventuel.

L'étape de stabilisation est préférablement mise en oeuvre sur un sirop hydrogéné déminéralisé, afin d'enlever toutes traces de nickel soluble ou d'autres catalyseurs d'hydrogénation.

On préfère soumettre au procédé conforme à l'invention un sirop de polyols obtenu par hydrogénation catalytique de sucres réducteurs simples ou complexes jusqu'à l'obtention d'un pourcentage de sucres réducteurs résiduels inférieur à 0,50 % mesuré selon la méthode de Bertrand. Ce pourcentage est plus préférentiellement inférieur à 0,25 % et plus préférentiellement encore inférieur à 0,20 %.

On préfère également utiliser un sirop de polyols déminéralisé, notamment dans le cas où l'étape de stabilisation consiste en un traitement fermentaire ou d'oxydation enzymatique.

C'est ainsi que l'on préfère utiliser comme produit destiné à l'étape de stabilisation, mais sans que cela soit pour autant impératif, un sirop de polyols présentant une densité optique de préférence inférieure ou égale à 0,200, plus préférentiellement inférieure ou égale à 0,170, et plus préférentiellement encore inférieure ou égale à 0,150 dans le test S.

Selon une première possibilité, l'étape de stabililisation consiste en une étape d'oxydation enzymatique par emploi d'une glucose oxydase. De préférence, cette oxydation enzymatique s'effectue en présence de catalase.

La glucose oxydase catalyse la réaction suivante :

Glucose + O₂ + H₂O → acide gluconique + H₂O₂

La catalase transforme l'eau oxygénée ainsi produite selon la réaction :

H₂O₂ → H₂O + 1/2 O₂

Une telle composition enzymatique est par exemple disponible auprès de la Société NOVO , DA-NEMARK sous la dénomination SP 358.

Cette oxydation enzymatique doit se dérouler dans un milieu aéré et le pH du milieu est maintenu à une valeur comprise entre 3,5 et 8,0, de préférence entre 4,0 et 7,0 et encore plus préférentiellement entre 5,0 et 6,0.

La concentration du sirop hydrogéné, préférentiellement déminéralisé, n'est pas critique et peut varier de 5 à 75 %. Toutefois, les concentrations élevées peuvent imposer de travailler en régulant le pH à l'aide d'une basé ou en effectuant l'oxydation en présence d'un sel tampon tel que le carbonate de calcium. La stabilisation du pH à une valeur comprise entre 5,0 et 6,0 est préférable.

Pour des raisons économiques, on préfère cependant effectuer l'oxydation sur des solutions aqueuses contenant environ 30 à 50 % de matières sèches. La température peut être ajustée dans une large gamme variant de 15 à 70°C mais pour des raisons de commodité on préfère travailler vers 30 - 40°C, températures auxquelles l'enzyme se montre la plus active.

Un matériel commode permettant d'effectuer cette oxydation consiste en un fermenteur aérobie, bien qu'il ne soit nullement nécessaire que cette étape se déroule dans des conditions stériles ni même rigoureuses d'aseptie. La quantité d'enzymes mise en oeuvre est telle que l'oxydation se déroule en 0,5 à 24 heures.

Cette étape d'oxydation enzymatique, en présence ou non de catalase, doit être suivie d'une étape de déminéralisation sur échangeur d'anions sous forme hydroxyle OH-, de façon à éliminer les acides formés par l'action de l'enzyme.

On préfère utiliser comme échangeur d'anions une résine anionique forte qui permet de fixer efficacement à la fois les acides faibles que sont l'acide gluconique ou d'autres acides d'oxydation du glucose qui ont pu apparaître, et les acides présents parfois de façon inhérente dans le produit hydrogéné comme l'acide citrique dans le cas du polydextrose hydrogéné.

Les résines préférées sont celles qui portent des groupements fonctionnels du type amine quaternaire et de préférence des groupements triméthylamine quaternaire telles que la résine AMBERLITE IRA 900 commercialisée par ROHM and HAAS.

Ces résines sont utilisées sous leur forme hydroxyle ou base forte OH-.

Pour augmenter leur rendement de régénération avec les alcalis, on peut préférer les coupler avec une résine anionique faible essentiellement porteuse de groupements aminés tertiaires telle que l'AMBERLITE IRA 93 de la même société.

Selon une seconde possibilité, l'étape de stabilisation consiste en une étape d'oxydation chimique. De préférence, cette oxydation chimique s'effectue en présence de bleu de méthylène, d'hydroquinone ou de résorcine de façon à catalyser les réactions et à augmenter les rendements d'oxydation. On peut procéder de façon connue pour les sirops de sucres réducteurs, par exemple en suivant le procédé SPENGLER - PFAN-NENSTIEL décrit dans le mémoire de DUBOURG et NAFFA (Bull. Soc. Chim. Fr. (1959) 1353 - 1362). Après oxydation, on doit purifier le sirop selon les mêmes techniques que celles citées ci-avant pour l'oxydation enzymatique.

Selon une troisième possibilité, l'étape de stabilisation est une étape fermentaire. On utilise alors la faculté de certains microorganismes, par exemple de certaines levures, de métaboliser les sucres simples et non les polyols et à les transformer par exemple en éthanol et en gaz carbonique. Il convient pour ce faire d'ajouter au sirop une source azotée comme par exemple des extraits de levures. Il va de soi que les conditions de fermentation doivent être ajustées en fonction du type de microorganisme choisi.

Toutefois, on préfère retenir des microorganismes à la fois osmophiles et thermophiles, de façon à pouvoir, pour des raisons de coût, travailler à haute matière sèche et à une température élevée.

On préfère également choisir, en vue de faciliter la purification ultérieure, des microorganismes non producteurs de métabolites secondaires. Seul alors du gaz carbonique ou des acides organiques sont produits.

Après fermentation, il convient d'éliminer la biomasse produite, par exemple par décantation, centrifugation ou filtration, avant de purifier davantage le sirop de polyols. Un chauffage vers 80°C permet d'éliminer l'éthanol éventuellement produit, alors qu'un traitement sur résines échangeuses d'anions s'avère utile pour éliminer des acides susceptibles de s'être formés lors de la fermentation.

Selon une quatrième et dernière méthode, l'étape de stabilisation consiste en une étape de dégradation alcaline ou étape de caramélisation.

On préfère travailler à un pH alcalin compris entre 8 et 12 et de préférence à chaud de façon à écourter les temps de réaction. Les produits de caramélisation, constitués essentiellement d'acides, peuvent être éliminés par passage sur résines.

Le procédé conforme à l'invention permet d'obtenir des compositions de polyols d'une stabilité thermique et chimique inégalée à ce jour, et ceci à un coût acceptable.

Bien entendu, dans tous les cas, les propriétés organoleptiques et la coloration résiduelle des produits obtenus peuvent encore être améliorées par des traitements complémentaires à l'aide par exemple de noir animal ou végétal.

L'un des principaux mérites de la présente invention est de fournir une composition de polyols très stable et non génératrice de goûts ou de colorations indésirables dans des conditions applicatives très diverses. L'invention se rapporte donc en troisième lieu à l'utilisation de ladite composition de polyols. Cette composition peut être utilisée en tant qu'agent édulcorant, agent de texture, agent complexant, agent humectant ou agent plastifiant, dans un grand nombre de produits. Elle peut avantageusement, compte tenu de sa totale compatibilité avec un grand nombre d'ingrédients et additifs utilisés habituellement dans l'industrie, être associée ou mélangée à des conservateurs, des émulsifiants, des arômes, des sucres, des édulcorants intenses, des bases, des principes actifs pharmaceutiques ou vétérinaires, des matières grasses, des agents de charge minéraux ou organiques tels que les polydextroses, les fibres, les fructooligosaccharides, les gommes, des agents gélifiants organiques ou minéraux tels que les protéines, les pectines, les celluloses modifiées, les extraits d'algues et de graines, les polysaccharides bactériens, les silices.

La composition de polyols conforme à l'invention peut convenir à la préparation de produits destinés à être ingérés par l'homme ou les animaux mais aussi entrer dans la formulation de produits capillaires ou d'application dermique. Elle peut également être employée dans l'industrie des détergents, des tabacs ou des plastiques. Ces produits, dans lesquels la composition de polyols est utilisable, peuvent présenter une texture liquide ou visqueuse : c'est le cas par exemple des boissons, des sirops, des émulsions, des suspensions, des élixirs, des bains de bouche, des ampoules buvables, des liquides pour vaisselle. Ils peuvent également présenter une texture pâteuse, comme les produits anti-acides ou les produits de confiserie non cristallisés ou semi-cristallisés tels que les bonbons, les gelées, les gommes, les pâtes à macher, les caramels, les chewing gums, les fourrages, les barres céréalières. Ils peuvent aussi présenter une texture gélifiée, comme c'est le cas des gels alimentaires tels que les flans, les confitures, les gelées, les desserts lactés, des gels pharmaceutiques et vétérinaires, ou des dentifrices. Ils peuvent enfin avoir une texture solide, comme dans les produits de pâtisserie, de biscuiterie, de panification, les comprimés, les entremets, les poudres atomisées ou extrudées d'édulcorants ou d'arômes, les lyophilisats pharmaceutiques ou vétérinaires, les tabacs et les poudres à laver le linge ou la vaisselle.

La composition conforme à l'invention est particulièrement recommandée pour préparer tous types de produits fabriqués en présence d'alcalis, tels que les mousses de polyuréthane, ou contenant des agents alcalins, tels que les anti-acides, les détergents, les mousses à raser, les crèmes épilatoires, et les dentifrices à base par exemple de carbonate ou de phosphate de sodium. Elle est également spécialement recommandée pour préparer des produits traités ou obtenus à très haute température. C'est le cas par exemple des sucres cuits.

Un autre avantage de la composition de polyols selon l'invention est d'être particulièrement stable vis-à-vis des enzymes de microorganismes ou vis-à-vis des arômes.

Les exemples qui suivent, et qui sont donnés à titre non limitatif, illustreront mieux l'invention.

### Exemple 1

On prépare une composition de polyols conforme à l'invention à partir d'un sirop de sorbitol commercialisé par la demanderesse sous le nom de marque NEOSORB®70/70. Ce sirop présente une densité optique dans le test S proche de 0,600.

Par dilution, on amène ce sirop à une matière sèche de 40 %. La solution obtenue est soumise à l'action de la glucose oxydase à raison de 70 unités GOX de glucose oxydase SP 358 par kilogramme de substrat sec. Cette réaction se déroule en cuve aérée à raison de 1,5 volume d'air par volume de solution et par minute, à un pH régulé à 5,0 par addition progressive de soude.

Elle a lieu à 35°C durant 16 heures, après quoi la solution est traitée sur une batterie de résines échangeuses d'ions comportant en série une résine cationique forte IR 200 C puis une résine anionique forte IRA 900.

Dans ces conditions, on obtient une composition de polyols présentant une densité optique très nettement abaissée, voisine de 0,070.

### Exemple 2 :

On prépare des dentifrices selon la formulation ci-dessous :
- bicarbonate de sodium 30,0 %
- composition de polyols (à 70 % MS) 36,0 %
- silice abrasive 8,0 %
- silice épaississante 5,0 %
- carboxyméthyl cellulose 0,7 %
- lauryl sulfate de sodium 1,7 %
- méthyl parabène 0,1 %
- oxyde de titane 0,7 %
- eau 17,8 %

Un premier dentifrice est fabriqué par emploi, en tant que composition de polyols, du sirop de sorbitol NEOSORB® 70/70 cité dans l'exemple 1 (dentifrice témoin).

Un second dentifrice est préparé en utilisant la composition de polyols selon l'invention décrite dans l'exemple 1.

On stocke les dentifrices emballés dans des tubes de type POLYFOIL^{R} pendant 10 jours à 45°C, ce qui correspond à une durée de stockage à 20°C de 15 mois environ.

On constate au terme de ce délai que le dentifrice témoin, initialement de couleur blanche, présente une couleur brun clair.

Par contre, le dentifrice préparé à partir de la composition de polyols conforme à l'invention, présente un aspect inchangé par rapport à l'état initial. Ceci constitue un avantage technique et commercial décisif pour les utilisateurs.

### Exemple 3:

On procède à un test de comparaison de stabilité thermique d'un sirop de sorbitol de l'art antérieur commercialisé par la demanderesse sous le nom de marque NEOSORB® 70/70 et de la composition de polyols selon l'invention obtenue selon la méthode décrite à l'exemple 1.

Pour cela, on soumet ces deux sirops ayant une matière sèche voisine de 70%, à un autoclavage à 117°C pendant 20 minutes.

On procède, après que les sirops soient revenus à température ambiante, à une dégustation en aveugle par un jury composé de 15 personnes.

Il apparaît de façon très nette que la composition de polyols conforme à l'invention est préférée, en raison de son goût très neutre, moins métallique et quasiment dépourvu d'une note caramel. Cette propriété rend la composition de polyols conforme à l'invention particulièrement intéressante pour de multiples applications.

De plus, il a été constaté une bien moindre interférence avec certaines substances et en particulier avec certains arômes et édulcorants intenses.

De ce fait, il est plus aisé que par l'utilisation de sirops de l'art antérieur, d'ajuster la qualité organoleptique de produits alimentaires, de dentifrices, de tabacs ou autres.

### Exemple 4 :

La réactivité vis-à-vis des arômes de spearmint et de peppermint d'un sirop de sorbitol selon l'art antérieur (NEOSORB®70/70 décrit à l'exemple 1) est comparée à la réactivité vis-à-vis des mêmes arômes d'une composition de sorbitol selon l'invention obtenue comme décrit à l'exemple 1.

Dans ce but, 40 parties de sirop de sorbitol (art antérieur ou invention) sont mélangées à 28 parties d'eau et à 0,8 part d'arôme de menthe. Les mélanges sont stockés dans des récipients hermétiques pendant 7 jours à 20°C, 40°C et 60°C.

Il apparaît, après cette période, selon les résultats obtenus par un panel de 15 personnes, que :
- en ce qui concerne le sirop de sorbitol de l'art antérieur, les échantillons maintenus à 20°C avec les arômes de spearmint ou de peppermint ont un goût mentholé bien préservé mais aussi un mauvais arrière-goût, les échantillons maintenus à 40°C ont une aromatisation mentholée dégradée, et les échantillons maintenus à 60°C n'ont plus de goût mentholé mais un goût complètement modifié,
- en ce qui concerne la composition de polyol selon l'invention, aucune modification en goût n'est notée à 20°C et 40°C et seulement une légère modification est perçue à 60°C.

Par analyse des arômes par chromatographie, il apparaît qu'après stockage dans les conditions ci-dessus, les mélanges avec le sirop de sorbitol selon l'art antérieur contiennent de nouveaux composants volatils en comparaison avec les arômes utilisés ou en comparaison aux mélanges contenant la composition de sorbitol conforme à l'invention.

En conclusion, la composition de sorbitol selon l'invention a une très faible réactivité aux arômes de menthe et est de ce fait plus intéressante pour l'industrie.

## Revendications

1. Utilisation pour la préparation de dentifrices d'une composition de polyols constituée de mélanges de polyols qui forment des sirops incristallisables à 20°C et à une matière sèche de 70% lorsqu'ils sont conservés dans un récipient hermétique à l'air durant un mois de stockage, présentant une teneur en sucres totaux après hydrolyse totale selon la méthode de Bertrand comprise entre 3,5 et 98%, et présentant une densité optique inférieure ou égale à 0,100 dans un test S consistant :
- à amener le sirop de polyol à tester à une matière sèche de 40% en poids,
- à ajouter à 5 ml de cette solution 500 mg d'hydrogénocarbonate de sodium de qualité ultrapure et 250 mg d'une solution aqueuse à 20% d'ammoniac,
- à mélanger l'ensemble et à chauffer pendant 2 heures au bain-marie à 100°C sans imposer d'agitation,
- à amener la solution à 20°C et à mesurer la densité optique de la solution ainsi obtenue à une longueur d'onde de 420 nm grâce à un spectromètre.

2. Utilisation selon la rev 1, **caractérisée par le fait que** la composition de polyols présente une densité optique inférieure ou égale à 0,075, de préférence inférieure à 0,060, et plus préférentiellement encore inférieure à 0,040 dans le test S.

3. Utilisation selon la revendication 1 ou la revendication 2, **caractérisée en ce que** la composition de polyols présente une teneur en sucres totaux après hydrolyse totale selon la méthode de Bertrand comprise entre 6 et 92% et de préférence entre 8 et 90%.

4. Utilisation selon l'une quelconque des revendications 1 à 3, **caractérisée en ce que** la composition de polyols comprend de 0,01 à 95% de mono- et/ou de disaccharides hydrogénés, le complément à 100 étant constitué d'oligo et polysaccharides hydrogénés.

5. Utilisation selon l'une quelconque des revendications 1 à 4, **caractérisée en ce que** dans la composition de polyols
- les monosaccharides hydrogénés sont choisis dans le groupe comprenant le sorbitol, l'iditol, le mannitol, le xylitol, l'arabitol et l'érythritol, et préférentiellement parmi le sorbitol, le mannitol et le xylitol,
- et les disaccharides hydrogénés sont choisis dans le groupe comprenant le maltitol, le maltulose hydrogéné, l'isomaltulose hydrogéné ou isomalt, l'isomaltitol, le lactitol, l'inulobiose hydrogéné et préférentiellement parmi le maltitol, le lactitol en l'isomaltulose hydrogéné.

## Patentansprüche

1. Verwendung einer Zusammensetzung von Polyolen, bestehend aus Gemischen von Polyolen, die bei 20 °C nicht kristallisierbare Sirupe bilden und nach einmonatiger Lagerung in einem luftdicht geschlossenen Behälter einen Gehalt an Trockensubstanz von 70 % aufweisen, wobei diese Zusammensetzung nach der Totalhydrolyse nach der Methode von Bertrand einen Gesamtzuckergehalt von 3,5 bis 98 % und eine optische Dichte kleiner oder gleich 0,100 in einem Test S aufweist, wobei der besagte Test S darin besteht:
- den zu untersuchenden Polyol-Sirup auf einen Gehalt an Trockensubstanz von 40 Gew.-% zu bringen,
- zu 5 ml dieser Lösung 500 mg Natriumhydrogencarbonat ultrareiner Qualität und 250 mg einer 20 %igen wässerigen Ammoniaklösung zu geben,
- das Ganze zu vermischen und im Wasserbad 2 Stunden ohne Rühren auf 100 °C zu erhitzen und
- die Lösung auf 20 °C abzukühlen und die optische Dichte der so erhaltenen Lösung bei einer Wellenlänge von 420 nm mit Hilfe eines Spektrometers zu bestimmen,
zur Herstellung von Zahnpasten.

2. Verwendung nach Anspruch 1, **dadurch gekennzeichnet, dass** die Zusammensetzung von Polyolen eine optische Dichte kleiner oder gleich 0,075, vorzugsweise kleiner 0,060 und noch bevorzugter kleiner 0,040 in dem Test S aufweist.

3. Verwendung nach Anspruch 1 oder 2, **dadurch gekennzeichnet, dass** die Zusammensetzung von Polyolen nach der Totalhydrolyse nach der Methode von Bertrand einen Gesamtzuckergehalt von 6 bis 92 % und vorzugsweise von 8 bis 90 % aufweist.

4. Verwendung nach einem der Ansprüche 1 bis 3, **dadurch gekennzeichnet, dass** die Zusammensetzung von Polyolen 0,01 bis 95 % hydrierte Mono- und/oder Disaccharide enthält, wobei die Differenz zu 100 % aus hydrierten Oligo- und Polysacchariden besteht.

5. Verwendung nach einem der Ansprüche 1 bis 4, **dadurch gekennzeichnet, dass** in der Zusammensetzung von Polyolen
- die hydrierten Monosaccharide unter Sorbitol, Iditol, Mannitol, Xylitol Arabitol und Erythritol und vorzugsweise unter Sorbitol, Mannitol und Xylitol ausgewählt werden und
- die hydrierten Disaccharide unter Maltitol, hydrierter Maltulose, hydrierter Isomaltulose oder Isomalt, Isomaltitol, Lactitol und hydrierter Inulobiose und vorzugsweise unter Maltitol, Lactitol und hydrierter Isomaltulose ausgewählt werden.

## Claims

1. Use, for the preparation of dentifrices, of a polyol composition which is constituted by mixtures of polyols forming syrups that are non-crystallizable at 20°C and at a solids content of 70% when they are stored in an air-tight container for a storage period of one month, having a total sugars content, after total hydrolysis according to the Bertrand method, of between 3.5 and 98% and exhibiting an optical density lower than or equal to 0.100 in an S test consisting of:
- bringing the polyol syrup to be tested to a solids content of 40% by weight,
- adding to 5 ml of this solution, 500 mg of sodium hydrogen carbonate of ultrapure quality and 250 mg of an aqueous solution containing 20% of ammonia,
- mixing the whole and heating it for 2 hours on a steam bath at 100°C without stirring being applied,
- bringing the solution to 20°C and measuring the optical density of the solution thus obtained at a wavelength of 420 nm using a spectrometer.

2. Use according to claim 1, **characterized in that** the polyol composition exhibits an optical density lower than or equal to 0.075, preferably lower than 0.060, and even more preferably lower than 0.040 in the S test.

3. Use according to claim 1 or claim 2, **characterized in that** the polyol composition has a total sugars content, after total hydrolysis according to the Bertrand method, of between 6 and 92% and preferably between 8 and 90%.

4. Use according to any one of claims 1 to 3, **characterized in that** the polyol composition comprises from 0.01 to 95% of hydrogenated mono- and/or disaccharides, the remainder to 100% consisting of hydrogenated oligo- and polysaccharides.

5. Use according to any one of claims 1 to 4, **characterized in that** in the polyol composition:
- the hydrogenated monosaccharides are selected from the group comprising sorbitol, iditol, mannitol, xylitol, arabitol and erythritol, and preferably from sorbitol, mannitol and xylitol,
- and the hydrogenated disaccharides are selected from the group comprising maltitol, hydrogenated maltulose, hydrogenated isomaltulose or isomalt, isomaltitol, lactitol, hydrogenated inulobiose, and preferably from maltitol, lactitol and hydrogenated isomaltulose.
